# EUROPÄISCHE PATENTANMELDUNG

(11) **EP 2 711 545 A1**
(43) Veröffentlichungstag der Anmeldung: **26.03.2014**
(21) Anmeldenummer: 12185019.2
(22) Anmeldetag: 19.09.2012
(51) Int. Cl.: F04B 1/02, F04B 1/04, F04B 23/06, F04B 53/10, F16K 15/04, A61B 17/3203

(54) **Pumpeinheit für die Wasserstrahlchirurgie**

(71) Anmelder: ERBE Elektromedizin GmbH, 72072 Tübingen (DE)
(72) Erfinder: Häbe, Elmar, 72534 Hayingen-Indelhausen (DE)
(74) Vertreter: Rüger, Barthelt & Abel

(57) **Zusammenfassung**

Die erfindungsgemäße Pumpeinheit (10) weist mindestens zwei Kolbenpumpen auf, zu denen Pumpzylinder (12, 13) und Pumpkolben (15, 16) gehören. Jede Kolbenpumpe umfasst mindestens ein Einlassventil (25, 27) und ein Auslassventil (34, 35). Mindestens die Einlassventile (25, 27), optional aber auch die Auslassventile (34, 36), sind als federlose Kugelrückschlagventile ausgebildet, bei denen sich die Ventilkugel (34) in einem Innenraum (39) befindet, der wenigstens an einer Seite (52) von einer Fläche des Gehäuses (vorzugsweise des ersten Gehäuseteils 11) begrenzt wird. Durch diese Bauform wird das eigentliche Rückschlagventil erst dann gefügt und somit vollständig hergestellt, wenn die Gehäuseteile (11, 17) zusammengeführt werden. Auf diese Weise lassen sich gut sterilisierbare und rückstandsfrei zu reinigende Rückschlagventile auf einfache und zuverlässige Weise in die Pumpeinheit (10) integrieren.

## Beschreibung

Die Erfindung betrifft eine Pumpeinheit, die insbesondere für die Wasserstrahlchirurgie vorgesehen ist.

Wasserstrahlchirurgie beruht auf der Applikation eines Strahls physiologischer Kochsalzlösung auf ein biologisches Gewebe, das dadurch vollständig oder teilweise durchtrennt und/oder aufgelöst wird. Beispielsweise können durch wasserstrahlchirurgische Eingriffe, je nach Prozessführung, sowohl Gewebeschnitte gesetzt werden, als auch z.B. Parenchymgewebe unter Schonung des durchsetzenden Gefäßsystems oder des Interstitiums aufgelöst werden. Damit sind bspw. nervenschonende Operationen möglich.

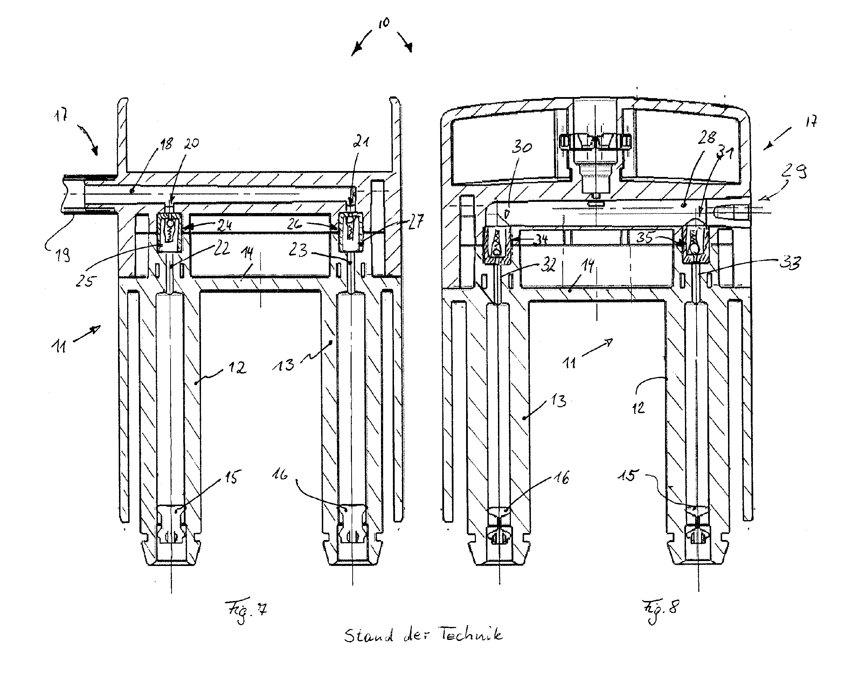

Zur Erzeugung eines entsprechenden Wasserstrahls muss das Behandlungsfluid, insbesondere NaCL-Lösung, mit einem gewünschten Druck und/oder mit einer gewünschten Fördermenge bereitgestellt werden, um dann über ein chirurgisches Instrument als Strahl appliziert zu werden. Zur Bereitstellung des Fluids dient im Stand der Technik eine Pumpeinheit, wie sie im Schnitt in den Figuren 7 und 8 veranschaulicht ist. Die dort gezeigte Pumpeinheit 10 ist als Einwegpumpe (d.h. zum einmaligen Gebrauch vorgesehenes Produkt) ausgebildet, die bedarfsweise in eine entsprechende Aufnahme eines medizinischen Geräts eingesetzt und mit diesem verrastet wird. Die Pumpeinheit 10 umfasst ein erstes Gehäuseteil 11 mit zwei zueinander achsparallel angeordneten Pumpzylindern 12, 13. Diese sind als runde, rohrartige Fortsätze ausgebildet, die sich von einem Kopfteil 14 weg erstrecken. In den Pumpzylindern 12, 13 sind Pumpkolben 15, 16 angeordnet, die abdichtend an der inneren Wandung der Pumpzylinder 12, 13 anliegen und längsverschiebbar angeordnet sind. An den distalen Enden der Pumpzylinder 12, 13 sowie an den Pumpkolben 15, 16 sind Verbindungsmittel, bspw. in Form umlaufender Nuten oder vorspringender Nasen oder dergleichen, angeordnet, um einerseits die Pumpeinheit 10 und damit die Pumpzylinder 12, 13 zu arretieren und andererseits die Pumpkolben 15, 16 gezielt hin- und hergehend zu bewegen.

Zu der Pumpeinheit 10 gehört ein zweiter Gehäuseteil 17, der fest mit dem ersten Gehäuseteil 11 verbunden ist. In dem zweiten Gehäuseteil 17 ist, wie aus Figur 7 ersichtlich, ein Ansaugkanal 18 ausgebildet, der z.B. über einen Schlauch 19 mit einem Fluidvorrat, bspw. physiologische Kochsalzlösung, verbunden wird.

Der Ansaugkanal 18 erstreckt sich quer zu den Pumpzylindern 12, 13 und weist jeweils eine zu dem jeweiligen Pumpzylinder 12, 13 hin führende Abzweigung 20, 21 auf. Ungefähr fluchtend zu diesen Abzweigungen 20, 21 ist in dem ersten Gehäuseteil 11, jeweils ein Einlasskanal 22, 23 ausgebildet. Zwischen der Abzweigung 20 und dem Einlasskanal 22 ist eine von beiden Gehäuseteilen 11, 17 begrenzte Kammer 24 festgelegt, in der ein Einlassventil 25 sitzt. Ebenso ist eine die Abzweigung 21 mit dem Eingangskanal 23 verbindende Kammer 26 vorgesehen, in der ein Einlassventil 27 sitzt.

Wie Figur 8 zeigt, weist der zweite Gehäuseteil 17 außerdem einen sich parallel zu dem Ansaugkanal 18 erstreckenden Druckkanal 28 auf, der an einer Seite des Gehäuseteils 17 an einem Anschluss 29 mündet. An diesem ist eine Druckleitung anzuschließen, die zu einem chirurgischen Instrument führt, das einen Fluidstrahl zur chirurgischen Behandlung eines Gewebes ausstößt.

Der Druckkanal 28 weist Abzweigungen 30, 31 auf, die mit Ausgangskanälen 32, 33 kommunizieren. Die Ausgangskanäle 32, 33 verbinden die Innenräume der Pumpzylinder 12, 13 über Ventile 34, 35 mit dem Druckkanal 28.

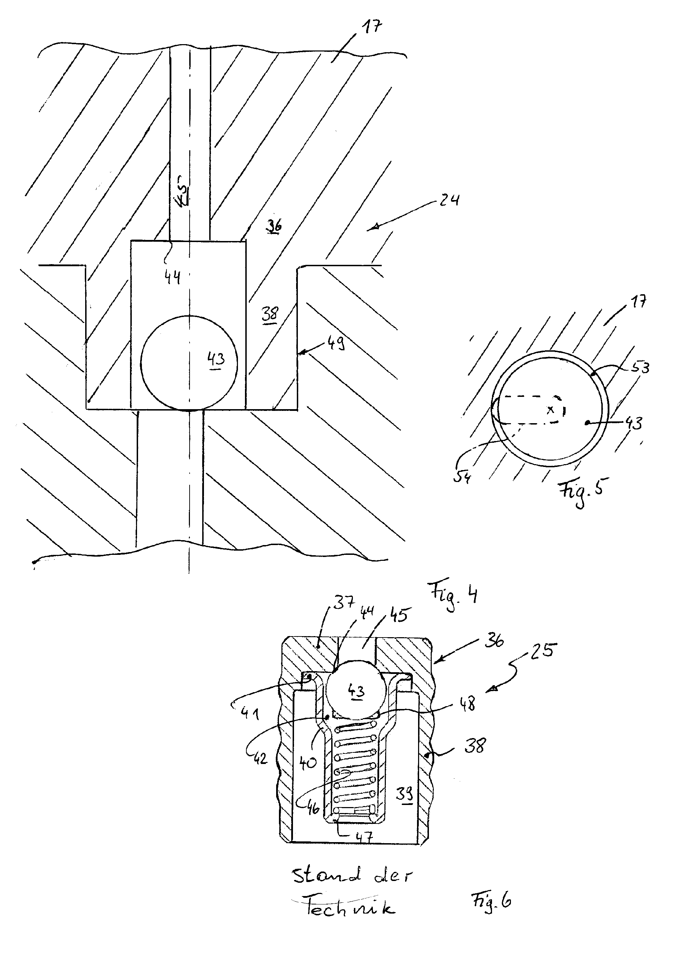

Bei der in den Figuren 7 und 8 dargestellten zum Stand der Technik gehörigen Pumpeinheit 10 sind die beiden Einlassventile 25, 27 und die Ventile 34, 35 untereinander gleich ausgebildet. Dazu ist das Einlassventil 25 in Figur 6 stellvertretend für alle Ventile 25, 27, 34, 35 beispielhaft dargestellt.

Das Ventil 25 ist ein federbelastetes Rückschlagventil. Es weist als äußeres Gehäuse eine Buchse 36 auf, die an einem Ende eine Endplatte 37 aufweist, von der sich ein rohrförmiger Fortsatz 38 weg erstreckt. In dem von dem Fortsatz 38 umschlossenen Raum 39 ist ein Innengehäuse 40 angeordnet. Dieses ist bspw. ein dünnwandiges Metallteil, das an einem auswärts gerichteten Flansch 41 z.B. im Presssitz mit der Buchse 36 verbunden ist. Das Innengehäuse 40 umschließt einen Innenraum 42, in dem eine Ventilkugel 43 angeordnet ist. Diese sitzt an dem Rand 44 einer die Endplatte 37 durchsetzenden Bohrung 45 und dichtet diese damit ab. Die Ventilkugel 43 wird durch eine Druckfeder 46 gegen den Rand 44 gedrückt, der somit einem Ventilsitz bildet. Die Druckfeder 46 stützt sich mit ihrem von der Ventilkugel 43 weg weisenden Ende bspw. an einem einwärts gebogenen Rand 47 eines distalen Endes des Innengehäuses 40 ab. Das Innengehäuse 40 weist außerdem mindestens ein seitliches Fenster 48 auf, das in Figur 6 weitgehend von der Ventilkugel 43 verdeckt ist und als Ausströmöffnung dient.

Derartige Pumpeinheiten haben sich in der Praxis grundsätzlich bewährt. Jedoch fordern sie einige Aufmerksamkeit bei ihrer Herstellung und Handhabung. Bei der Pumpeneinheit handelt es sich um ein medizinisches Sterilprodukt. Dies erfordert, dass bereits im Herstellungsprozess, der in einem Reinraum stattfindet, die verwendeten Komponenten, beispielsweise die Ventile, einem definierten Reinheitsgrad entsprechen. In Bezug auf die im Stand der Technik verwendeten Ventile bedeutet dies, dass Sie frei von Prozessstoffen, wie beispielsweise Öle und Fette, und frei von Reinigungsmitteln sind. Dieser Reinigungsprozess ist bei Ventilen, bei denen ein Federmittel die Ventilkugel in den Ventilsitz presst, fast nicht oder nur unter erschwerten Bedingungen möglich. Dieser Reinigungsaufwand ist enorm.

Nach der Herstellung bzw. der Montage der Pumpeneinheit muss für eine vollständige Sterilisierung dieser Pumpeinheit 10 gesorgt werden. Dazu kann die Pumpeneinheit einem Gassterilisationsverfahren (z.B. mit Ethylenoxid) unterzogen werden. Hierzu muss jedoch mindestens eine Seite der jeweiligen Ventilpaarung (Auslass/Einlass) für das Gas passierbar sein, damit dieses in alle relevanten Hohlräume eindringen kann. Bei einer Verwendung von federbelasteten Ein- und Auslassventilen ist diese Passierbarkeit nicht hinreichend gegeben..

Eine weitere Anforderung an solch eine Pumpeneinheit ist die unmittelbare Funktionsfähigkeit bzw. Einsatzfähigkeit nach deren Lagerung. Die Lagerzeit nach der sterilen Herstellung kann bis zu 3 Jahre betragen. Die Einlassventile von Pumpeneinheiten, die aus einem Lager entnommen werden müssen schon durch das Anliegen eines geringen Unterdrucks/Vakuum von maximal -350 mbar öffnen. Aufgrund unterschiedlicher physikalischer Einflüsse, wie beispielsweise Verklemmen oder Festsitzen der Kugel im Kugelsitz, ist dies bei den dem Stand der Technik entsprechenden, federbelasteten Einlassventilen nicht immer gegeben, was dann eine Nichtfunktion der Pumpeneinheit zur Folge hat.

Es sind deshalb auch schon Pumpeinheiten 10 in Gebrauch gekommen, bei denen die Druckfedern 46 der beiden Einlassventile 25, 27 fehlen. Diese Pumpeneinheiten haben sich in der Praxis bewährt. Ein Festsitzen der Einlassventile ist bei diesen Pumpen nicht mehr vorgekommen und die Durchlässigkeit für das zur Sterilisation verwendeten Gas ist vollständig gegeben. Jedoch macht sich bei diesen Pumpeinheiten eine gewisse Funktionsbeeinträchtigung dahingehend bemerkbar, dass die Zuleitung nun vollständig entlüftet sein muss, um die Erstbefüllung der Pumpe sicherstellen zu können. Zusätzlich wurde festgestellt, dass die Förderleistung bei sehr niedrigen Effektstufen, also bei sehr geringen Kolbengeschwindigkeiten, nicht mehr sichergestellt wird. Bei niedrigen Effektstufen ist der vom Pumpenkolben 15, 16 geförderte Fluidstrom gering und reicht bei den dem Stand der Technik entsprechenden Ventilen nicht aus, um ein korrektes Schaltverhalten der Ventile zu gewährleisten.
Davon ausgehend, ist es Aufgabe der Erfindung, eine Pumpeinheit anzugeben, die einen verbesserten Einsatzbereich aufweist.

Diese Aufgabe wird mit der Pumpeinheit nach Anspruch 1 gelöst:

Die erfindungsgemäße Pumpeinheit zeichnet sich ausgehend von der vorbekannten Pumpeinheit durch eine besondere Ausbildung zumindest der Einlassventile aus. Diese sind federlos und ohne Innengehäuse ausgebildet. Die Ventilverschlusselemente in Gestalt von Ventilkugeln sind in dem Innenraum des Ventils frei beweglich angeordnet und nicht in einem gesonderten Innengehäuse gefangen. Sie bewegen sich lose und liegen an keiner Stelle des Innenraums mit Vorspannung an. Es können dadurch zwei die Ventilkugel betreffende Maßnahmen getroffen werden. Die Ventilkugel kann einen vergrößerten Durchmesser erhalten und sie kann aus einem Material mit verminderter Dichte gearbeitet werden. Ein Haften oder Kleben der Ventilkugel an inneren Oberflächen, insbesondere am Ventilsitz, sicher ausgeschlossen und die Mitnahme der Ventilkugel durch bewegte Luft schon beim Ansaugen der Flüssigkeit wird wesentlich erleichtert. Das trägt dazu bei, dass die Pumpeinheit zu Beginn ihres Einsatzes auch bei langsamen Kolbenbewegungen Behandlungsfluid sicher ansaugt. Nachdem dies auch bei sehr geringen Effektstärken sichergestellt werden kann, bei denen sich die Pumpkolben mit äußerst geringer Geschwindigkeit bewegen, ist der Einsatzbereich der Pumpeinheit zu geringen Effektstärken hin erweitert.

Die Vorteile hinsichtlich der Erweiterung des Einsatzbereichs ergeben sich aus der Umgestaltung, zumindest der als Einlassventile genutzten Rückschlagventile. Das Einlassventil wird erst bei der Montage der Gehäuseteile geschlossen. Vormontierte Ventile, in deren wenig zugänglichen Innenräumen Fremdstoffe vorhanden sein könnten, kommen als Einlassventile nicht zum Einsatz. Der Eintrag von Verschmutzungen kann so besser kontrolliert werden. Außerdem können sie durch den Reinigungsprozess besser und somit restlos beseitigt werden. Reinigungsrückstände, d.h. Rückstände von Reinigern, die beim Reinigungsprozess herkömmlicher vormontierter Ventile in das Ventil eingebracht werden, können vermindert oder vermieden werden. Außerdem wird durch die zumindest einseitige Begrenzung des Ventilinnenraums durch den ersten Gehäuseteil sicher ausgeschlossen, dass Fremdstoffe in Gestalt von Herstellungsprozessrückständen, wie z.B. Zieh- und Schmierfetten in den Innenraum gelangen oder dort verbleiben.

Das Verhältnis zwischen Kugeldurchmesser und Ventilsitz kann anders als bisher gewählt werden. Die Kugel kann einen Durchmesser erhalten, der relativ groß im Vergleich zu dem Ventilsitzdurchmesser ist. Bei einer bevorzugten Ausführungsform beträgt der Kugeldurchmesser das 1,5-fache des Durchmessers des Ventilsitzes. Geringfügige kleinere Kugeldurchmesser oder auch größere Kugeldurchmesser sind möglich. Durch diese Maßnahme und durch den Wegfall einer Ventilschließfeder wird ein Haften der Kugel, d.h. eine Kohäsion durch das dichte Fügen der Kugel und des Ventilsitzes vermieden. Außerdem wird das Öffnen und Schließen der Kugel durch die Kräfte des Fluidstroms gefördert. Durch den Wegfall des Ventilgehäuses steht mehr Bauraum für die Ventilkugel zur Verfügung. Diese kann somit einen vergrößerten Durchmesser erhalten. Sie bietet dem Fluidstrom dadurch mehr Oberfläche und wird so leichter in Öffnungsrichtung sowie Schließrichtung bewegt. Dies gilt insbesondere für Fluidströme mit sehr langsamen Fließgeschwindigkeiten, also bei sehr geringen Effektstärken.

Während der Innenraum, in dem sich die Ventilkugel frei bewegen kann, an mindestens einer Seite, bspw. einer Stirnseite, z.B. der zuflussseitigen Stirnseite, von dem ersten Gehäuseteil, abgeschlossen ist, kann der Innenraum ansonsten von Flächen des zweiten Gehäuseteils oder von einer in das Gehäuseteil eingesetzten Buchse umgeben sein. Der Ventilsitz kann an der Buchse oder auch dem zweiten Gehäuseteil ausgebildet sein.

Dieses Konzept eröffnet außerdem die besonders einfache übersichtliche Montagemöglichkeit mit offen liegenden Flächen vor dem Fügen der beiden Gehäuseteile. Damit lassen sich leicht anspruchsvolle Reinheitsstandards einhalten.

Bei einer bevorzugten Ausführungsform weist ein Gehäuseteil einen Rohrfortsatz auf, der in eine Tasche des anderen Gehäuseteils ragt. Damit kann ein druckfester, fluiddichter Presssitz gebildet werden, der von dem in dem Ventil herrschenden Druck in einer die Dichtwirkung unterstützenden Weise belastet wird. Hat der Rohrfortsatz unter dem wirkenden Innendruck eine Aufweitungstendenz, wird der Rohrfortsatz an die Wandung der Tasche noch zusätzlich angepresst.

Der Ausgangskanal ist vorzugsweise dem Eingangskanal gegenüberliegend angeordnet. Dadurch ergibt sich für die Ventilkugel auch bei geringen Fluiddurchsätzen eine Anströmung, die die Ventilkugel sicher in Öffnungs- oder Schließrichtung bewegt. Insbesondere ist der Innenraum frei von jeglichen Teilen oder mechanischen Mitteln, ausgenommen der Ventilkugel. Dadurch entstehen an keiner Stelle des Innenraums Strömungen, die quer zur Bewegungsrichtung der Ventilkugel wirken. Somit wird eine Anströmung der Ventilkugel quer zur Fließrichtung des Mediums und quer zur Längsbewegung der Ventilkugel vermieden. Dadurch kann der Fluidstrom ungehindert, also optimal auf die Ventilkugel wirken und diese in Strömungsrichtung mitnehmen was das präzise Öffnen und Schließen des Ventils ermöglicht. Störungen durch Querströmungen des Mediums können verhindert werden.

Vorzugsweise wird der Ausgang des Innenraums des Ventils von einer Öffnung mit einer zu der Ventilkugel nicht passenden Kante umschlossen, so dass die Öffnung von der Ventilkugel nicht verschlossen werden kann. Eine zu der Ventilkugel nicht passende Kante ist jede Kante, die sich nicht lückenlos an eine Kugeloberfläche anschmiegt. Es kann sich somit um eine Ovalöffnung, eine von einem Steg durchquerte runde oder anderweitig geformte Öffnung, eine neben einem Fortsatz oder einem Stift angeordnete Öffnung oder dergleichen, handeln. Dadurch kann die Ventilkugel ungeachtet der herrschenden Strömungsgeschwindigkeiten und Drücke den Ausgang nicht verschließen.

Vorzugsweise ist der Innenraum, in dem sich die Ventilkugel befindet, im Wesentlichen zylindrisch ausgebildet. Die Ventilkugel legt dann mit der zylindrischen Wandfläche einen Ringspalt fest. Dieser hat vorzugsweise eine Querschnittsfläche, die geringer ist als die Querschnittsfläche des Eingangs. Vorzugsweise ist das Verhältnis der Querschnittsfläche des Ringspalts zu der Querschnittsfläche des Eingangs zwischen 0,1 und 1 festgelegt. Vorzugsweise liegt es zwischen 0,2 und 0,4 - bei der konkreten Ausführungsform beträgt es bevorzugterweise 0,3. Der Durchmesser der Ventilkugel kann z.B. zwischen 2 mm und 5 mm, vorzugsweise bei 3 mm liegen. Sie hat vorzugsweise eine geringe Dichte (z.B. weniger als 3 g/cm³). Sie besteht vorzugsweise aus einem Kunststoff oder Keramik. Sie kann eine Hohlkugel sein.

Weiter bevorzugt sind die beiden Gehäuseteile durch Klemm- und Rastmittel miteinander verbunden. Die Klemm- und Rastverbindung kann ohne Klebstoffe und/oder Dichtmittel auskommen, wodurch sich ein sauberer, technologisch leicht beherrschbarer Aufbau ergibt.

Weitere Einzelheiten von vorteilhaften Ausführungsformen der Erfindung sind Gegenstand der Beschreibung, der Zeichnung oder von Unteransprüchen. Es zeigen:
Figur 1 und 2 die erfindungsgemäße Pumpeinheit, in verschiedenen Querschnittsdarstellungen,
Figur 3 ein Einlassventil der Pumpeinheit nach den Figuren 1 und 2, in schematisierter vergrößerter Darstellung (nicht maßstäblich),
Figur 4 eine alternative Ausführungsform des Einlassventils der erfindungsgemäßen Pumpeinheit, in Längsschnittdarstellung,
Figur 5 das Einlassventil nach Figur 4, in Querschnittsdarstellung,
Figur 6 ein Ventil einer Pumpeneinheit nach dem Stand der Technik, in längs geschnittener Darstellung, und
Figur 7 und 8 eine Pumpeinheit nach dem Stand der Technik, in längs geschnittener Darstellung.

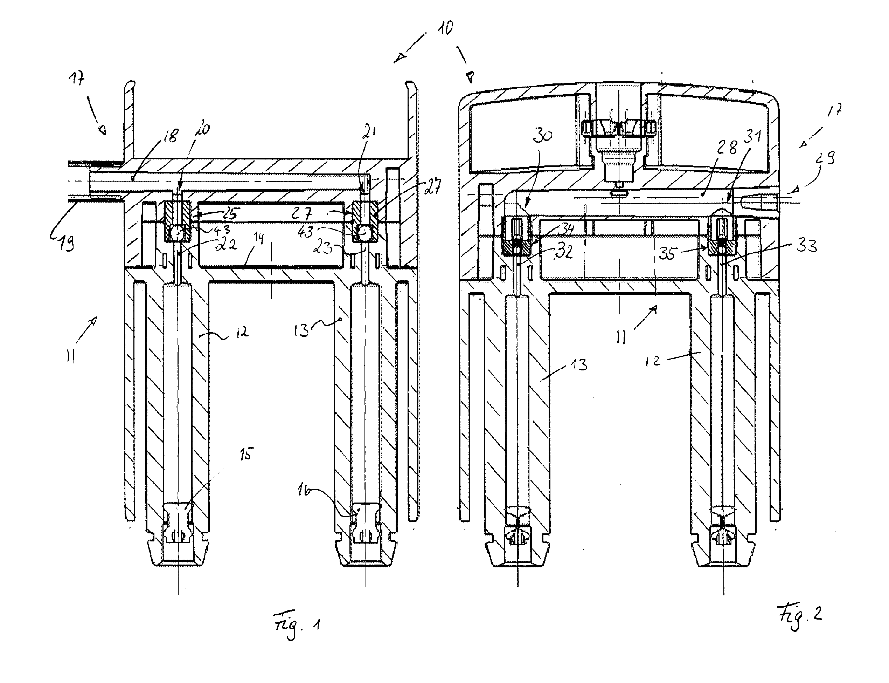

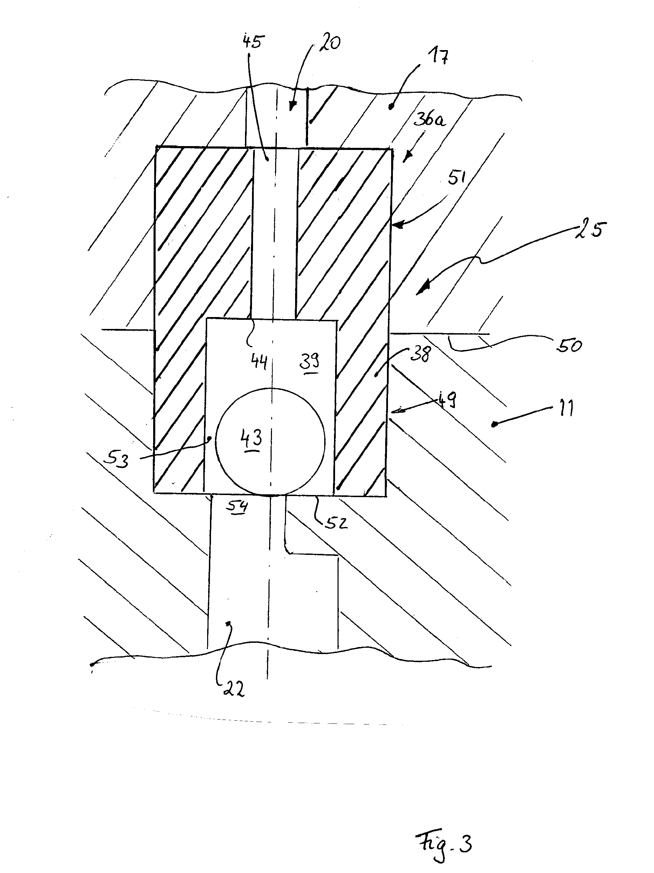

In Figur 1 und 2 ist eine Pumpeinheit 10 veranschaulicht, die bis auf den Aufbau ihrer Einlassventile 25, 27 der Pumpeinheit 10 nach dem Stand der Technik entspricht, wie er eingangs im Zusammenhang mit den Figuren 6 bis 8 beschreiben worden ist. Es werden deshalb, soweit bauliche und/oder funktionelle Übereinstimmung besteht, in den Figuren 1 bis 5 die im Zusammenhang mit den Figuren 6 bis 8 eingeführten Bezugszeichen in gleicher Bedeutung gebraucht. Die zu den Figuren 6 bis 8, insbesondere den Figuren 7 und 8, gegebene Beschreibung des Aufbaus und der Funktion gilt mit Ausnahme der nachfolgend beschriebenen Einzelheiten für die erfindungsgemäßen Ausführungsformen entsprechend: Die Einlassventile 25, 27 sind im Aufbau untereinander gleich und in Figur 3 am Beispiel des Einlassventils 25 schematisch veranschaulicht. Die Beschreibung desselben gilt entsprechend für das Einlassventil 27.

Wie aus Figur 3 ersichtlich weist der erste Gehäuseteil 11 eine Tasche 49 auf, die von einer Trennfuge 50 ausgeht, bei der der erste Gehäuseteil 11 und der zweite Gehäuseteil 17 aneinander anschließen. Die Tasche 49 ist beispielsweise zylindrisch ausgebildet. Eine ähnliche Tasche 51 ist in dem zweiten Gehäuseteil 17 ausgebildet und so angeordnet, dass die beiden Taschen 49, 51 miteinander fluchten und die Kammer 24 bilden.

In die Tasche 51 ist eine Buchse 36a eingepresst oder auf andere Weise darin verankert. Diese Buchse 36a besteht z.B. aus Metall. Sie kann auch aus anderen Materialien z.B. Kunststoff oder Keramik bestehen. Sie enthält die als Einlasskanal dienende Bohrung 45, die mit der Abzweigung 20 fluchtet. An ihrem der Ventilkugel 43 zugewandten Ende weitet sich die Bohrung 45 um einen Raum zur Aufnahme der Ventilkugel 43 zu bilden. Im Übergang ist der Ventilsitz 44 ausgebildet. Ein den Innenraum 39 umgebender Teil 38 der Buchse 36a bildet den Fortsatz, der in die Tasche 49 ragt. Vorzugsweise ist dieser Teil der Buchse 36, d.h. der Fortsatz 38, leicht konisch ausgebildet oder außen mit Ringrippen versehen (ähnlich Figur 6), um mit der Wandung der Tasche 49 des ersten Gehäuseteils 11 einen fluiddichten Sitz zu definieren.

Der Innenraum 39 wird somit an seiner an den Eingangskanal 22 anschließenden Seite 52 von einer Fläche des ersten Gehäuseteils 11 begrenzt. Im Übrigen wird er von der inneren Wandung der Buchse 36a begrenzt. In dem Innenraum 39 ist die Ventilkugel 43 in einer durch eine strichpunktierte Linie angedeutete Bewegungsrichtung frei beweglich.

Der Durchmesser der Ventilkugel 43 ist geringer als der Durchmesser des Innenraums 39. Dadurch ist ein z.B. aus der Figur 5 ersichtlicher Ringspalt 53 festgelegt, dessen Querschnittsfläche vorzugsweise kleiner ist als die Querschnittsfläche der Bohrung 45. Die Querschnittsfläche des Ringspalts 53 beträgt Pi/4 mal die Differenz aus dem Quadrat des Durchmessers des Innenraums 39 und dem Quadrat des Kugeldurchmessers der Ventilkugel 43. Die Querschnittsfläche der Bohrung 45 beträgt Pi/4 mal dem Quadrat des Durchmessers der Bohrung 45.

Der durch den Rand 44 gebildete Ventilsitz liegt einer Ausgangsöffnung 54 gegenüber, die so angeordnet oder ausgebildet ist, dass sich die Kugel 43 nicht verschließend vor die Ausgangsöffnung 54 legen kann. Der Rand der Ausgangsöffnung 54 passt nicht auf die Oberfläche der Ventilkugel 43. Dies kann z.B. durch außermittige Anordnung der Ausgangsöffnung 54 erreicht werden, wie es in Figur 3 angedeutet ist. Außerdem kann die Ausgangsöffnung 54 von der Kreisform abweichen, wie es Figur 5 zeigt. Die Anordnung und die Form der Ausgansöffnung 54 begrenzen den Weg, den die Ventilkugel 43 zurücklegen kann. Dadurch kann der Schaltweg der Ventile beeinflusst werden. Je geringer der Weg ist den die Kugel zum Schließen des Ventilsitzes benötigt, desto schneller kann das Ventil geschlossen werden wodurch der Rückfluss des Mediums auf das Minimum reduziert werden kann.

Figur 4 veranschaulicht eine abgewandelte Ausführungsform des Einlassventils 25. Bei dieser wird auf eine gesonderte Buchse 36a verzichtet. Vielmehr ist die Buchse 36a ein einstückiger, nahtlos mit dem zweiten Gehäuseteils 17 verbundener Teil 36 und somit ein Bestandteil des zweiten Gehäuseteils 17 und besteht aus demselben Stoff wie dieses. Im Ergebnis ist der rohrförmige Fortsatz 38 Bestandteil des zweiten Gehäuseteils 17 und erstreckt sich vorzugsweise klemmend, d.h. im Presssitz, in die Tasche 49. Ansonsten gilt die vorige Beschreibung entsprechend.

Die insoweit beschriebene erfindungsgemäße Pumpeinheit 10 arbeitet wie folgt:

Die Pumpeinheit 10 wird in ein medizinisches Gerät eingefügt, in dem Stellantriebe vorhanden sind, die mit den Pumpkolben 15, 16 koppeln. Es wird der Schlauch 19 angeschlossen, der mit einem Reservoir der gewünschten Flüssigkeit bspw. NaCl-Lösung verbunden wird. Außerdem wird an den Anschluss 29 eine Leitung angeschlossen, die zu einem chirurgischen Instrument mit Strahlejektionsdüse führt. Es werden dann die Stellantriebe für die Kolben 15, 16 aktiviert, die sich gegenläufig bewegen. Die leichtgängigen Einlassventile 25, 27 geben dabei jeweils beim Kolbenrückhub (Ansaughub) den Weg frei, und sie schließen, wenn sich der betreffende Kolben 15 oder 16 in Richtung auf das Einlassventil 25, 27 hin bewegt. Der Ringspalt 53 ist dabei so relativ eng bemessen, dass die Ventilkugel 43 auch dann in Schließrichtung bewegt wird, wenn noch keine Flüssigkeit, sondern nur Luft durch das jeweilige Einlassventil 25, 27 strömt. Unabhängig von der Größe des Ringspalts 53 trägt dazu auch schon die Größe und das vorzugsweise geringe Gewicht der Ventilkugel 43 bei. Diese besteht vorzugsweise aus einem Kunststoff. Sie kann als Vollkugel oder als Hohlkugel ausgebildet sein. Sie kann auch aus anderen Stoffen, insbesondere Keramik bestehen. Durch die Gestaltung des Einlassventils 25 gemäß mindestens einer der oben beschriebenen Maßnahmen wird das Ansaugen von Fluid auch dann erleichtert und ermöglicht, wenn sich der betreffende Kolben 15 oder 16 nur langsam bewegt.

Sobald die angesaugte Flüssigkeit den jeweiligen Pumpzylinder 12, 13 füllt, wird beim jeweiligen Kompressionshub Fluid mit der gewünschten Geschwindigkeit durch das jeweilige Auslassventil 34, 35 in das chirurgische Instrument gepumpt. Die Bewegung der Kolben 15, 16 kann so aufeinander abgestimmt werden, dass dies unterbrechungsfrei geschieht.

Die erfindungsgemäße Pumpeinheit 10 weist mindestens zwei Kolbenpumpen auf, zu denen runde oder auch anderweitig gestaltete Pumpzylinder 12, 13 und passende Pumpkolben 15, 16 gehören. Jede Kolbenpumpe umfasst mindestens ein Einlassventil 25, 27 und ein Auslassventil 34, 35. Mindestens die Einlassventile 25, 27, optional aber auch die Auslassventile 34, 36, sind als federlose Kugelrückschlagventile ausgebildet, bei denen sich die Ventilkugel 34 in einem Innenraum 39 befindet, der wenigstens an einer Seite 52 von einer Fläche des Gehäuses (vorzugsweise des ersten Gehäuseteils 11) begrenzt wird. Durch diese Bauform wird das eigentliche Rückschlagventil erst dann gefügt und somit vollständig hergestellt, wenn die Gehäuseteile 11, 17 zusammengeführt werden. Auf diese Weise lassen sich gut sterilisierbare und rückstandsfrei zu reinigende Rückschlagventile auf einfache und zuverlässige Weise in die Pumpeinheit 10 integrieren.

### Bezugszeichenliste:

- 10: Pumpeinheit
- 11: erster Gehäuseteil
- 12, 13: Pumpzylinder
- 14: Kopfteil
- 15, 16: Pumpkolben
- 17: zweiter Gehäuseteil
- 18: Ansaugkanal
- 19: Schlauch
- 20, 21: Abzweigung
- 22, 23: Eingangskanal
- 24: Kammer
- 25: Einlassventil
- 26: Kammer
- 27: Einlassventil
- 28: Druckkanal
- 29: Anschluss
- 30, 31: Abzweigung
- 32, 33: Ausgangskanäle
- 34, 35: Auslassventile
- 36, 36a: Buchse, Teil
- 37: Endplatte
- 38: Fortsatz, Teil
- 39: Innenraum
- 40: Innengehäuse
- 41: Flansch
- 42: Innenraum
- 43: Ventilkugel
- 44: Rand, Ventilsitz
- 45: Bohrung, Einlasskanal
- 46: Druckfeder
- 47: Rand
- 48: Fenster

- 49: Tasche
- 50: Trennfuge
- 51: Tasche
- 52: Seite
- 53: Ringspalt
- 54: Ausgangsöffnung

## Patentansprüche

1. Pumpeinheit (10), insbesondere für die Wasserstrahlchirurgie,
mit einem Pumpengehäuse, das einen ersten Gehäuseteil (11) und einen zweiten Gehäuseteil (17) aufweist,
wobei der erste Gehäuseteil (11) wenigstens zwei Pumpzylinder (12, 13) aufweist, die zur Aufnahme von Pumpkolben (15, 16) eingerichtet sind,
wobei der zweite Gehäuseteil (17) einen Ansaugkanal (18) und einen Druckkanal (28) aufweist,
wobei zwischen den Pumpzylindern (12, 13) und dem Ansaugkanal (18) Einlassventile (25, 27) sowie zwischen den Pumpzylindern (12, 13) und dem Druckkanal (28) Auslassventile (34, 35) wirksam sind,
wobei jedes Einlassventil (25, 27) einen Innenraum (39) aufweist, in dem jeweils eine Ventilkugel (43) angeordnet ist, wobei der Innenraum (39) einen Einlasskanal (45) und eine Ausgangsöffnung (54) aufweist,
**dadurch gekennzeichnet,**
**dass** der Innenraum (39) an zumindest einer Stelle (52) von einem der Gehäuseteile (11, 17) und an einer anderen Stelle von einem anderen Teil (36, 38) begrenzt ist und
**dass** die Ventilkugel (43) in dem Innenraum (39) frei beweglich gelagert ist.

2. Pumpeinheit nach Anspruch 1, **dadurch gekennzeichnet, dass** der andere Teil (38) das zweite Gehäuseteil (17) oder ein in dem zweiten Gehäuseteil (17) sitzendes Buchsenteil (36) ist.

3. Pumpeinheit nach Anspruch 2, **dadurch gekennzeichnet, dass** der Einlasskanal (45) an dem zweiten Gehäuseteil (17) oder dem Buchsenteil (36) angeordnet ist und den Ventilsitz (44) bildet.

4. Pumpeinheit nach Anspruch 3, **dadurch gekennzeichnet, dass** der zweite Gehäuseteil (17) oder der Buchsenteil (36) einen Fortsatz (38) bilden, der in eine Tasche (49) des ersten Gehäuseteils (11) ragt.

5. Pumpeinheit nach Anspruch 4, **dadurch gekennzeichnet, dass** der Fortsatz (38) und das erste Gehäuseteil (11) einen fluiddichten Presssitz bilden.

6. Pumpeinheit nach einem der vorstehenden Ansprüche, **dadurch gekennzeichnet, dass** der Ausgang durch eine einzige Öffnung (54) gebildet ist, die dem Einlasskanal (45) bezüglich der Ventilkugel (43) diametral gegenüberliegend angeordnet ist.

7. Pumpeinheit nach Anspruch 6, **dadurch gekennzeichnet, dass** die Ausgangsöffnung (54) von einer zu der Ventilkugel (43) nicht passenden Kante umgeben ist.

8. Pumpeinheit nach einem der vorstehenden Ansprüche, **dadurch gekennzeichnet, dass** der Innenraum (39) eine zylindrische Wandfläche aufweist, die mit der Ventilkugel (43) einen Ringspalt (53) festlegt.

9. Pumpeinheit nach Anspruch 8, **dadurch gekennzeichnet, dass** der Ringspalt (53) eine Querschnittsfläche (A_{ring}) festlegt und dass der Einlasskanal (45) eine Querschnittsfläche (A_{eingang}) festlegt, so dass das Verhältnis der Querschnittsfläche (A_{ring}) des Ringspalts (53) zu der Querschnittsfläche (A_{eingang}) des Einlasskanal (45) zwischen 0,1 und 1, vorzugsweise zwischen 0,2 und 0,4 liegt.

10. Pumpeinheit nach einem der vorstehenden Ansprüche, **dadurch gekennzeichnet, dass** zur Verbindung der Gehäuseteile (11, 17) untereinander Klemmmittel und/oder Rastmittel vorgesehen sind.
